# EUROPEAN PATENT SPECIFICATION

(11) **EP 3 380 604 B1**
(45) Date of publication and mention of the grant of the patent: **28.12.2022**
(21) Application number: 16798512.6
(22) Date of filing: 21.11.2016
(51) Int. Cl.: C12N 5/10, C12N 7/02, C12N 7/04, C12N 15/85, C12N 15/86

(54) **STABLE CELL LINES FOR RETROVIRAL PRODUCTION**
STABILE ZELLLINIEN FÜR DIE RETROVIRENPRODUKTION
LIGNEES CELLULAIRES STABLES POUR LA PRODUCTION DE RETROVIRUS

(30) Priority: 24.11.2015 GB 201520761; 26.05.2016 GB 201609303
(43) Date of publication of application: 03.10.2018
(60) Divisional application: 19151187.2
(73) Proprietor: GlaxoSmithKline Intellectual Property Development Limited, Brentford Middlesex TW8 9GS (GB)
(72) Inventor: JOHNSON, Sabine, Stevenage Hertfordshire SG1 2NY (GB); PALLANT, Celeste, Stevenage Hertfordshire SG1 2NY (GB); VAMVA, Eirini, Stevenage Hertfordshire SG1 2NY (GB); VINK, Conrad, Stevenage Hertfordshire SG1 2NY (GB)
(74) Representative: Wilkinson, Johanna Elise
(86) International application number: PCT/EP2016/078336
(87) International publication number: WO 2017/089308

(56) References cited:
- WO-A1-00/66758
- WO-A1-00/66758
- WO-A1-00/66758
- WO-A1-01/91802
- WO-A1-01/91802
- WO-A1-95/03400
- WO-A1-95/03400
- WO-A1-95/03400
- WO-A1-2008/099148
- WO-A1-2008/099148
- WO-A1-2017/089307
- WO-A1-2017/089307
- WO-A2-02/072836
- WO-A2-02/072836
- WO-A2-02/072836
- GB-A- 2 538 321
- GB-A- 2 538 321
- GB-A- 2 538 321
- GB-A- 2 538 324
- GB-A- 2 538 324
- GB-A- 2 538 324
- M. Niebert ET AL: "Characterization of Chromosomally Assigned Replication-Competent Gamma Porcine Endogenous Retroviruses Derived from a Large White Pig and Expression in Human Cells", JOURNAL OF VIROLOGY., vol. 76, no. 6, 15 March 2002 (2002-03-15) , pages 2714-2720, XP055332042, US ISSN: 0022-538X, DOI: 10.1128/JVI.76.6.2714-2720.2002
- YAJIN NI ET AL: "Generation of a packaging cell line for prolonged large-scale production of high-titer HIV-1-based lentiviral vector", JOURNAL OF GENE MEDICINE, JOHN WILEY & SONS, INC, US, vol. 7, no. 6, 1 June 2005 (2005-06-01), pages 818-834, XP002664828, ISSN: 1099-498X, DOI: 10.1002/JGM.726 [retrieved on 2005-02-03]
- Félix Recillas-Targa: "Mammalian Gene Transfer and Expression 337 MOLECULAR BIOTECHNOLOGY Multiple Strategies for Gene Transfer, Expression, Knockdown, and Chromatin Influence in Mammalian Cell Lines and Transgenic Animals", , 1 January 2006 (2006-01-01), XP055338237, Retrieved from the Internet: URL:http://rd.springer.com/content/pdf/10. 1385/MB:34:3:337.pdf [retrieved on 2019-06-27]
- KHALED S. SANBER ET AL: "Construction of stable packaging cell lines for clinical lentiviral vector production", SCIENTIFIC REPORTS, vol. 5, no. 1, 12 March 2015 (2015-03-12), XP055491335, DOI: 10.1038/srep09021
- MARIA MERCEDES SEGURA ET AL: "New developments in lentiviral vector design, production and purification", EXPERT OPINION ON BIOLOGICAL THERAPY, vol. 13, no. 7, 16 April 2013 (2013-04-16) , pages 987-1011, XP055340869, ASHLEY, LONDON; GB ISSN: 1471-2598, DOI: 10.1517/14712598.2013.779249
- M. NIEBERT ET AL: "Characterization of Chromosomally Assigned Replication-Competent Gamma Porcine Endogenous Retroviruses Derived from a Large White Pig and Expression in Human Cells", JOURNAL OF VIROLOGY., vol. 76, no. 6, 15 March 2002 (2002-03-15) , pages 2714-2720, XP055332042, US ISSN: 0022-538X, DOI: 10.1128/JVI.76.6.2714-2720.2002
- YAJIN NI ET AL: "Generation of a packaging cell line for prolonged large-scale production of high-titer HIV-1-based lentiviral vector", JOURNAL OF GENE MEDICINE, JOHN WILEY & SONS, INC, US, vol. 7, no. 6, June 2005 (2005-06), pages 818-834, XP002664828, ISSN: 1099-498X, DOI: 10.1002/JGM.726 [retrieved on 2005-02-03] cited in the application
- Félix Recillas-Targa: "Mammalian Gene Transfer and Expression 337 MOLECULAR BIOTECHNOLOGY Multiple Strategies for Gene Transfer, Expression, Knockdown, and Chromatin Influence in Mammalian Cell Lines and Transgenic Animals", Molecular Biotechnology, vol. 34, no. 3 November 2006 (2006-11), November 2006 (2006-11), pages 337-357, XP055338237, Retrieved from the Internet: URL:http://rd.springer.com/content/pdf/10. 1385/MB:34:3:337.pdf [retrieved on 2017-01-23]
- M. Niebert ET AL: "Characterization of Chromosomally Assigned Replication-Competent Gamma Porcine Endogenous Retroviruses Derived from a Large White Pig and Expression in Human Cells", JOURNAL OF VIROLOGY., vol. 76, no. 6, 15 March 2002 (2002-03-15) , pages 2714-2720, XP055332042, US ISSN: 0022-538X, DOI: 10.1128/JVI.76.6.2714-2720.2002
- YAJIN NI ET AL: "Generation of a packaging cell line for prolonged large-scale production of high-titer HIV-1-based lentiviral vector", JOURNAL OF GENE MEDICINE, JOHN WILEY & SONS, INC, US, vol. 7, no. 6, 1 June 2005 (2005-06-01), pages 818-834, XP002664828, ISSN: 1099-498X, DOI: 10.1002/JGM.726 [retrieved on 2005-02-03]
- Félix Recillas-Targa: "Mammalian Gene Transfer and Expression 337 MOLECULAR BIOTECHNOLOGY Multiple Strategies for Gene Transfer, Expression, Knockdown, and Chromatin Influence in Mammalian Cell Lines and Transgenic Animals", , 1 January 2006 (2006-01-01), XP055338237, Retrieved from the Internet: URL:http://rd.springer.com/content/pdf/10. 1385/MB:34:3:337.pdf [retrieved on 2019-06-27]
- KHALED S. SANBER ET AL: "Construction of stable packaging cell lines for clinical lentiviral vector production", SCIENTIFIC REPORTS, vol. 5, no. 1, 12 March 2015 (2015-03-12), XP055491335, DOI: 10.1038/srep09021
- MARIA MERCEDES SEGURA ET AL: "New developments in lentiviral vector design, production and purification", EXPERT OPINION ON BIOLOGICAL THERAPY, vol. 13, no. 7, 16 April 2013 (2013-04-16) , pages 987-1011, XP055340869, ASHLEY, LONDON; GB ISSN: 1471-2598, DOI: 10.1517/14712598.2013.779249

## Description

### FIELD OF THE INVENTION

The invention relates to nucleic acid vectors comprising genes required for retroviral production and uses thereof. Also provided are methods of making retroviral packaging/producer cell lines comprising the nucleic acid vectors as described herein.

### BACKGROUND TO THE INVENTION

In gene therapy, genetic material is delivered to endogenous cells in a subject in need of treatment. The genetic material may introduce novel genes to the subject, or introduce additional copies of pre-existing genes, or introduce different alleles or variants of genes that are present in the subject. Viral vector systems have been proposed as an effective gene delivery method for use in gene therapy (Verma and Somia (1997) Nature 389: 239-242).

In particular, these viral vectors are based on members of the retrovirus family due to their ability to integrate their genetic payload into the host's genome. Retroviral vectors are designed to keep the essential proteins required for packaging and delivery of the retroviral genome, but any non-essential accessory proteins including those responsible for their disease profile are removed. Examples of retroviral vectors include lentiviral vectors, such as those based upon Human Immunodeficiency Virus Type 1 (HIV-1), which are widely used because they are able to integrate into non-proliferating cells.

Currently, the majority of viral vectors are produced by transient co-transfection of viral genes into a host cell line. The viral genes are introduced using bacterial plasmids which exist in the host cell for only a limited period of time because the viral genes remain on the plasmids and are not integrated into the genome. As such, transiently transfected genetic material is not passed on to subsequent generations during cell division.

There are several drawbacks associated with transient transfection, such as batch-to-batch variability, the high cost of transfection reagents and the difficulty to maintain quality control (see Segura et al. (2013) Expert Opin. Biol. Ther. 13(7): 987-1011). The process of transfection itself is also labour-intensive and challenging to scale up. There is also the difficult task of removing plasmid impurities which are carried over during vector preparation (see Pichlmair etal. (2007) J. Virol. 81(2): 539-47).

In order to address problems associated with transient transfection, there has been a desire to develop retroviral packaging and producer cell lines in order to simplify retroviral vector production.

Packaging cell lines have been generated by transfecting a cell line capable of packaging retroviral vectors with plasmids, where individual plasmids carry the retroviral packaging genes and unique eukaryotic selection markers. The packaging genes are integrated into the packaging cell line's genome and are described as being stably transfected. Over the past 20 years various attempts have been made to generate stable packaging and producer cell lines for retroviral vectors.

WO2012/028681 describes methods to make semi-stable lentiviral packaging cell lines using a hybrid baculo-Adeno associated virus (AAV) vector. WO2003/064665 describes a rev-independent lentiviral production system for producing a lentivirus-derived vector particle.

There have been many reported problems in the packaging and producer cell lines produced via integration of retroviral vector components into the host cell genome. In the first instance, sequential introduction of retroviral vector components can be laborious and inflexible. There have also been problems with genetic and/or transcriptional instability of retroviral vector components when they are integrated into the host cell genome because the site of integration is unpredictable (Ni et al. (2005) J. Gene Med. 7: 818-834.). A significant drop in viral vector productivity has also been reported during suspension adaptation and scale-up of the producer cell lines (Farson et al. (2001) Hum. Gene Ther. 12: 981-997; Guy et al. (2013) Hum. Gene Ther. Methods. 24(2): 125-39).

It is therefore an object of the present invention to provide a method of making stable retroviral packaging and producer cell lines which overcomes one or more of the disadvantages associated with existing methods.

### SUMMARY OF THE INVENTION

The present inventors have developed a new way of making packaging and producer cell lines which involves the use of nucleic acid vectors comprising a non-mammalian origin of replication and the ability to hold at least 25 kilobases (kb) of DNA, such as bacterial artificial chromosomes, comprising the retroviral genes essential for retroviral vector production. This allows expression of the retroviral genes required for production of replication defective retroviral vector particles to ameliorate problems associated with transient transfection methods.

The use of a nucleic acid vector comprising a non-mammalian origin of replication and which has the ability to hold at least 25 kb of DNA (*i.e*. large-construct DNA) has several advantages. In the first instance, the vectors can first be manipulated in non-mammalian cells (*e.g*. microbial cells, such as bacterial cells) rather than mammalian host cells which makes them much easier to use (*e.g.* bacterial artificial chromosomes can first be manipulated in *E*. *coli*). Once the nucleic acid vector has been prepared, it can be introduced into a mammalian host cell and any mammalian cells which have the nucleic acid vector integrated into the endogenous chromosomes can be selected in order to isolate a stable cell line.

Introduction of the retroviral nucleic acids into the mammalian host cell also occurs in a single step which helps to reduce selection pressure and silencing timeframe. This allows for faster screening of potential packaging cells and reduces the cost of materials because only a single vector is used, rather than previous methods which use multiple plasmid vectors. In particular, use of the current system reduces the cost of manufacture by saving on plasmid costs, transfection reagents required (*e.g*. Polyethylenimine [PEI]), reducing the amount of Benzonase treatment required (there is a reduced amount of DNA in the lentiviral harvest, therefore less Benzonase is needed to remove the excess in downstream processing) and reduced cost of testing (there is no need to test for residual plasmid in the lentiviral product).

Furthermore, the retroviral genes essential for retroviral production (with our without the transfer vector) are present within the nucleic acid vector so that when the vector is introduced into mammalian host cells, all of the retroviral genes incorporated in the nucleic acid vector will integrate at one locus within the endogenous mammalian host cell genome. This can overcome problems such as gene silencing which can occur when the retroviral genes are integrated randomly and at different loci within the host cell genome.

The use of nucleic acid vectors of the invention therefore provides advantages in the generation of retroviral packaging and producer cell lines.

In one aspect of the invention, there is provided a nucleic acid vector comprising a non-mammalian origin of replication, one or a plurality of recombination sites(s) and the ability to hold at least 25 kilobases (kb) of DNA, selected from: a bacterial artificial chromosome (BAC), a yeast artificial chromosome (YAC), a P1-derived artificial chromosome, a fosmid or a cosmid. comprising retroviral nucleic acid sequences encoding:
gag and pol proteins, and
an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus
wherein each of the retroviral nucleic acid sequences are arranged as individual expression constructs within the nucleic acid vector.

In another aspect of the invention, there is provided a method of producing a stable retroviral packaging cell line, comprising:
(a) introducing a nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid. comprising retroviral nucleic acid sequences encoding:
   gag and pol proteins, and
   a env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus,
   wherein each of the retroviral nucleic acid sequences has its own promoter within the nucleic acid vector,into a culture of mammalian host cells; and
(b) selecting within the culture for a mammalian host cell which has the nucleic acid sequences encoded on the vector integrated into an endogenous chromosome of the mammalian host cell.

According to a further aspect of the invention, there is provided a method of producing a replication defective retroviral vector particle, comprising:
(a) introducing the nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid. comprising retroviral nucleic acid sequences encoding:
   gag and pol proteins, and
   an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus,
   wherein each of the retroviral nucleic acid sequences has its own promoter within the nucleic acid vector,into a culture of mammalian host cells;
(b) selecting within the culture for a mammalian host cell which has the nucleic acid sequences encoded on the vector integrated into an endogenous chromosome of the mammalian host cell; and
(c) further culturing the mammalian host cell under conditions in which the replication defective retroviral vector particle is produced.

### BRIEF DESCRIPTION OF THE FIGURES

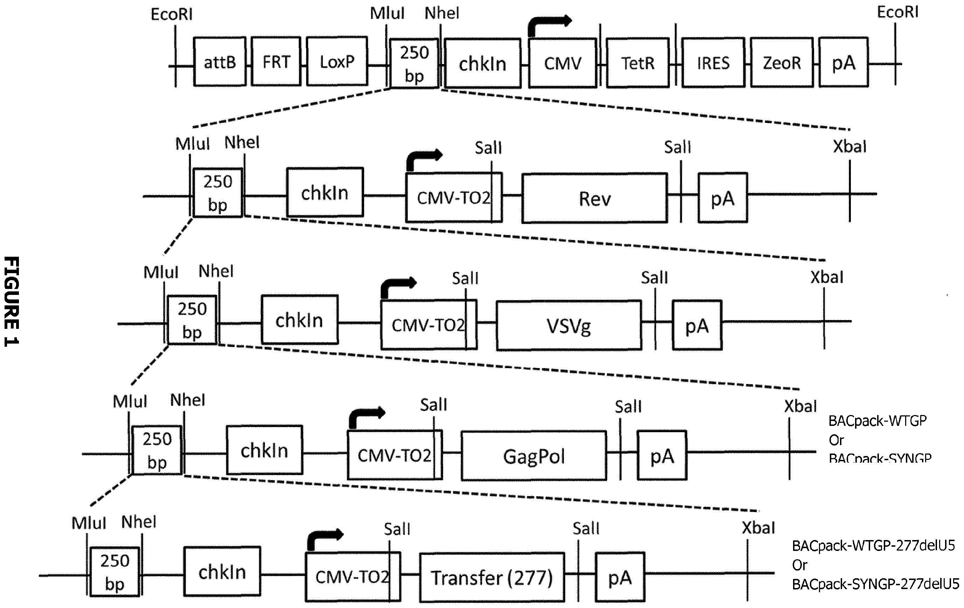

**FIGURE 1****: A stepwise guide to the construction of BACpack-WTGP-277deIU5 and BACpack-SYNGP-277deIU5.**

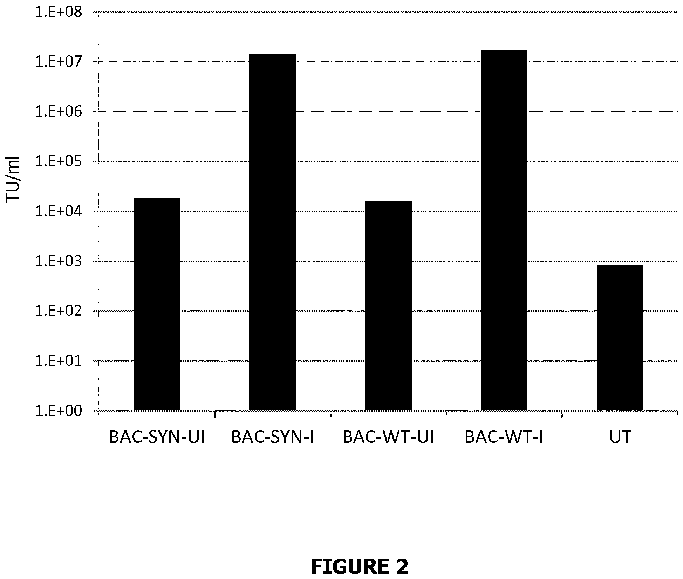

**FIGURE 2****: Selection of a stable polyclonal pool.** HEK293T adherent cells were transfected with BACpackWTGagPol-Transfer using Calcium Phosphate. Stable pools were generated after 2 weeks' Zeocin selection. To see whether the stable transfectants were capable of generating virus, the stable poly-pools were induced with Doxycycline for 48 hours. Viral supernatant was harvested 48 hours post induction, filtered through a 0.22µm filter and titrated by transducing HEK293T cells. GFP positive transduced cells were used to calculate the Transducing Units/ml (TU/mL).

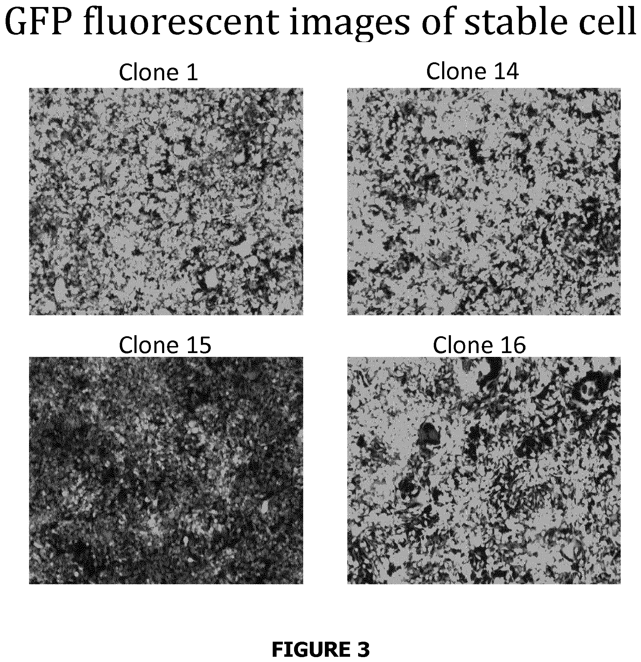

**FIGURE 3****: Generating stable transfection suspension clones.** HEK293 6E cells were transfected with BACpackWTGagPol-Transfer using 293fectin reagent. Stable pools were generated after 2 weeks' Zeocin selection. The stable pools were cloned by limiting dilution into 96 well plates to obtain single cell clones, which were subsequently expanded. GFP detected by fluorescence microscopy of the best clones 1, 14, 15 and 16, obtained with adherent medium (DMEM + FBS) followed by suspension adaptation (FreeStyle medium).

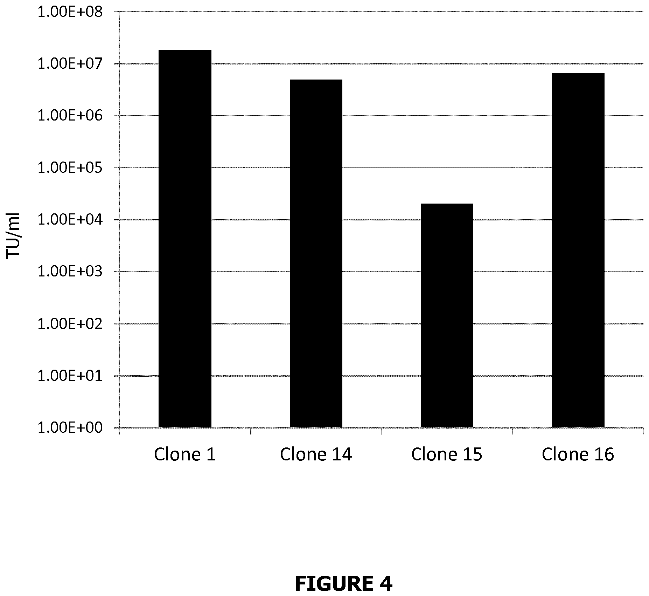

**FIGURE 4****: Induction of Lentivirus in the suspension clones.** To see whether the stable HEK6E transfectants were capable of generating virus, 20 ml of the stable suspension clones were induced with Doxycycline (2µg/ml) for 48 hours. Viral supernatant was harvested 48 hours post induction, filtered through a 0.45µm filter and titrated by transducing HEK293T cells. GFP positive transduced cells were used to calculate the Transducing Units/ml (TU/mL).

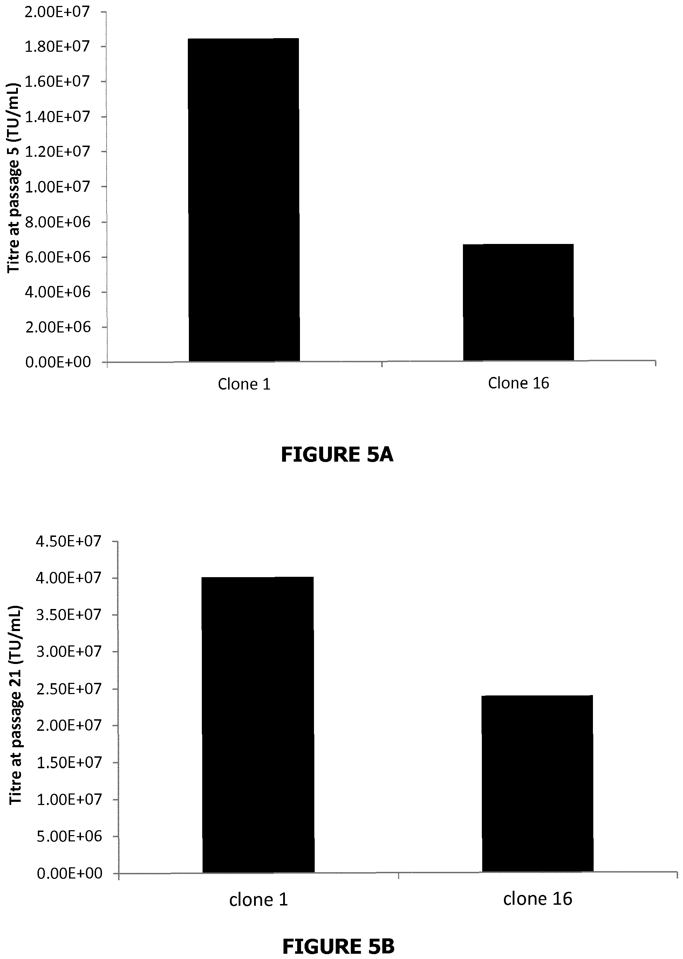

**FIGURE 5****: Vector titres of clones produced according to Example 4.** Results show vector titres from clones 1 and 16 increased modestly between passage 5 and passage 21.

### DETAILED DESCRIPTION OF THE INVENTION

### DEFINITIONS

Unless defined otherwise, all technical and scientific terms used herein have the same meaning as is commonly understood by one of skill in the art to which this invention belongs.

The term "comprising" encompasses "including" or "consisting" e.g. a composition "comprising" X may consist exclusively of X or may include something additional e.g. X + Y.

The term "consisting essentially of" limits the scope of the feature to the specified materials or steps and those that do not materially affect the basic characteristic(s) of the claimed feature.

The term "consisting of" excludes the presence of any additional component(s).

The term "about" in relation to a numerical value x means, for example, x ± 10%, 5%, 2% or 1%.

The term "vector" or "nucleic acid vector" refers to a vehicle which is able to artificially carry foreign (*i.e*. exogenous) genetic material into another cell, where it can be replicated and/or expressed. Examples of vectors include non-mammalian nucleic acid vectors, such as bacterial artificial chromosomes (BACs), yeast artificial chromosomes (YACs), P1-derived artificial chromosomes (PACs), cosmids or fosmids.

Other examples of vectors include viral vectors, such as retroviral and lentiviral vectors, which are of particular interest in the present application. Lentiviral vectors, such as those based upon Human Immunodeficiency Virus Type 1 (HIV-1) are widely used as they are able to integrate into non-proliferating cells. Viral vectors can be made replication defective by splitting the viral genome into separate parts, *e.g*., by placing on separate plasmids. For example, the so-called first generation of lentiviral vectors, developed by the Salk Institute for Biological Studies, was built as a three-plasmid expression system consisting of a packaging expression cassette, the envelope expression cassette and the vector expression cassette. The "packaging plasmid" contains the entire *gag-pol* sequences, the regulatory (*tat* and *rev*) and the accessory (*vif, vpr, vpu, nef*) sequences. The "envelope plasmid" holds the Vesicular stomatitis virus glycoprotein (VSVg) in substitution for the native HIV-1 envelope protein, under the control of a cytomegalovirus (CMV) promoter. The third plasmid (the "transfer plasmid") carries the Long Terminal Repeats (LTRs), encapsulation sequence (ψ), the Rev Response Element (RRE) sequence and the CMV promoter to express the transgene inside the host cell.

The second lentiviral vector generation was characterized by the deletion of the virulence sequences *vpr, vif, vpu and nef.* The packaging vector was reduced to *gag, pol, tat* and rev genes, therefore increasing the safety of the system.

To improve the lentiviral system, the third-generation vectors have been designed by removing the *tat* gene from the packaging construct and inactivating the LTR from the vector cassette, therefore reducing problems related to insertional mutagenesis effects.

The various lentivirus generations are described in the following references: First generation: Naldini etal. (1996) Science 272(5259): 263-7; Second generation: Zufferey et al. (1997) Nat. Biotechnol. 15(9): 871-5; Third generation: Dull etal. (1998) J. Virol. 72(11): 8463-7, A review on the development of lentiviral vectors can be found in Sakuma etal. (2012) Biochem. J. 443(3): 603-18 and Picanço-Castro et al. (2008) Exp. Opin. Therap. Patents 18(5):525-539.

The term "non-mammalian origin of replication" refers to a nucleic acid sequence where replication is initiated and which is derived from a non-mammalian source. This enables the nucleic acid vectors of the invention to stably replicate and segregate alongside endogenous chromosomes in a suitable host cell (*e.g*. a microbial cell, such as a bacterial or yeast cell) so that it is transmittable to host cell progeny, except when the host cell is a mammalian host cell. In mammalian host cells, nucleic acid vectors with non-mammalian origins of replication will either integrate into the endogenous chromosomes of the mammalian host cell or be lost upon mammalian host cell replication. For example, nucleic acid vectors with non-mammalian origins of replication such as bacterial artificial chromosomes (BAC), P1-derived artificial chromosome (PAC), cosmids or fosmids, are able to stably replicate and segregate alongside endogenous chromosomes in bacterial cells (such as *E. coli*), however if they are introduced into mammalian host cells, the BAC, PAC, cosmid or fosmid will either integrate or be lost upon mammalian host cell replication. Yeast artificial chromosomes (YAC) are able to stably replicate and segregate alongside endogenous chromosomes in yeast cells, however if they are introduced into mammalian host cells, the YAC will either integrate or be lost upon mammalian host cell replication. Therefore, in this context, the nucleic acid vectors of the invention act as reservoirs of DNA (*i.e*. for the genes essential for retroviral production) which can be easily transferred into mammalian cells to generate stable cell lines for retroviral production. Examples of non-mammalian origins of replication include bacterial origins of replications, such as *oriC oriV* or *oriS,* or yeast origins of replication, also known as Autonomously Replicating Sequences (ARS elements).

The nucleic acid vectors of the present invention comprise a non-mammalian origin of replication and are able to hold at least 25 kilobases (kb) of DNA. In one embodiment, the nucleic acid vector has the ability to hold at least 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340 or 350 kb of DNA. It will be understood that references to "ability to hold" has its usual meaning and implies that the upper limit for the size of insert for the nucleic acid vector is not less than the claimed size (*i.e.* not less than 25 kb of DNA).

The aim of the present invention is to include the genes essential for retroviral packaging in a single construct (*i.e*. the nucleic acid vector). Therefore, the nucleic acid vectors of the invention, must be able to hold large inserts of DNA. For the avoidance of doubt, it will be understood that references to "nucleic acid vectors" or "artificial chromosomes" do not refer to natural bacterial plasmids (*e.g*. such as the plasmids currently used in transient transfection methods) because these are not able to hold at least 25 kb of DNA. The maximum size insert which a plasmid can contain is about 15 kb. Such nucleic acid vectors also do not refer to bacteriophages which generally only hold maximum inserts of 5-11 kb. Therefore, in one embodiment the nucleic acid vector of the invention is not a plasmid, bacteriophage or episome.

The term "endogenous chromosomes" refers to genomic chromosomes found in the host cell prior to generation or introduction of an exogenous nucleic acid vector, such as a bacterial artificial chromosome.

The terms "transfection", "transformation" and "transduction" as used herein, may be used to describe the insertion of the non-mammalian or viral vector into a target cell. Insertion of a vector is usually called transformation for bacterial cells and transfection for eukaryotic cells, although insertion of a viral vector may also be called transduction. The skilled person will be aware of the different non-viral transfection methods commonly used, which include, but are not limited to, the use of physical methods (*e.g*. electroporation, cell squeezing, sonoporation, optical transfection, protoplast fusion, impalefection, magnetofection, gene gun or particle bombardment), chemical reagents (*e.g*. calcium phosphate, highly branched organic compounds or cationic polymers) or cationic lipids (*e.g*. lipofection). Many transfection methods require the contact of solutions of plasmid DNA to the cells, which are then grown and selected for a marker gene expression.

The term "promoter" refers to a sequence that drives gene expression. In order to drive a high level of expression, it may be beneficial to use a high efficiency promoter, such as a non-retroviral, high efficiency promoter. Examples of suitable promoters may include a promoter such as the human cytomegalovirus (CMV) immediate early promoter, spleen focus-forming virus (SFFV) promoter, Rous sarcoma virus (RSV) promoter, or human elongation factor 1-alpha (pEF) promoter.

A Tet operon (Tetracycline-Controlled Transcriptional Activation) may be used in a method of inducible gene expression, wherein transcription is reversibly turned on or off in the presence of the antibiotic tetracycline or one of its derivatives (*e.g*. doxycycline). In nature, the Ptet promoter expresses TetR, the repressor, and TetA, the protein that pumps tetracycline antibiotic out of the cell. In the present invention, the Tet operon may be present or absent, for example, in one embodiment the Tet operon may be present in the promoter.

The term "selectable marker" refers to a gene that will help select cells actively expressing an inserted gene (*e.g*. a transgene). Examples of suitable selection markers include, enzymes encoding resistance to an antibiotic (*i.e.* an antibiotic resistance gene), *e.g.,* kanamycin, neomycin, puromycin, hygromycin, blasticidin, or zeocin. Another example of suitable selection markers are fluorescent proteins, for example green fluorescent protein (GFP), red fluorescent protein (RFP) or blue fluorescent protein (BFP).

The term "polyA signal" refers to a polyadenylation signal sequence, for example placed 3' of a transgene, which enables host factors to add a polyadenosine (polyA) tail to the end of the nascent mRNA during transcription. The polyA tail is a stretch of up to 300 adenosine ribonucleotides which protects mRNA from enzymatic degradation and also aids in translation. Accordingly, the nucleic acid vectors of the present invention may include a polyA signal sequence such as the human beta globin or rabbit beta globin polyA signals, the simian virus 40 (SV40) early or late polyA signals, the human insulin polyA signal, or the bovine growth hormone polyA signal. In one embodiment, the polyA signal sequence is the human beta globin polyA signal.

The term "intron sequence" refers to a nucleotide sequence which is removed from the final gene product by RNA splicing. The use of an intron downstream of the enhancer/promoter region and upstream of the cDNA insert has been shown to increase the level of gene expression. The increase in expression depends on the particular cDNA insert. Accordingly, the nucleic acid vector of the present invention may include introns such as human beta globin intron, rabbit beta globin intron II or a chimeric human beta globin-immunoglobulin intron. In one embodiment, the intron is a human beta globin intron and/or a rabbit beta globin intron II.

The term "packaging cell line" refers to a cell line with stably inserted gag and pol protein and envelope glycoprotein genes. Alternatively, the term "producer cell line" refers to a packaging cell line with a stably inserted transfer vector containing a transgene of interest. It will be understood by a person skilled in the art that the nucleic acid vectors described herein may be used to generate packaging cell lines (*i.e.* when at least the *gag, pol* and *env* genes are present on the nucleic acid vector and incorporated into a host cell) or producer cell lines (*i.e*. when the nucleic acid vector additionally comprises the transfer vector components to be incorporated into a host cell along with the *gag, pol* and *env* genes).

The term "stably transfected" refers to cell lines which are able to pass introduced retroviral genes to their progeny (*i.e.* daughter cells), either because the transfected DNA has been incorporated into the endogenous chromosomes or via stable inheritance of exogenous chromosomes.

### NUCLEIC ACID VECTORS

According to one aspect of the invention, there is provided a nucleic acid vector comprising a non-mammalian origin of replication, one or a plurality of recombination sites(s) and the ability to hold at least 25 kilobases (kb) of DNA, selected from: a bacterial artificial chromosome (BAC), a yeast artificial chromosome (YAC), a P1-derived artificial chromosome, a fosmid or a cosmid. comprising retroviral nucleic acid sequences encoding:
gag and pol proteins, and
an env protein, obtained from or derived from a virus selected from a Vesiculovirus, Lyssavirus, Arenavirus, Alphavirus, Filovirus, Coronavirus, Respirovirus, Respiratory syncytia virus, Hepacivirus, Influenzavirus and Nucleopolyhedrovirus
wherein, each of the retroviral nucleic acid sequences has its own promoter within the nucleic acid vector.

Current methods for generating retroviral vectors involve transient transfection of the retroviral genes into a host cell. However, many disadvantages have been associated with this method because it is costly, laborious and difficult to scale-up. One solution would be to engineer a packaging cell line that stably incorporates the retroviral packaging genes to avoid the problems associated with transient transfection and to reduce variable retroviral vector output.

The present inventors have found that nucleic acid vectors described herein can be used to generate a retroviral packaging cell line which ameliorates previous difficulties associated with retroviral vector production methods. For example, known methods of producing retroviral packaging cell lines involve multiple rounds of selection after each retroviral gene is introduced. This process can take up to six months and is heavily labour intensive. By including all of the retroviral genes in the nucleic acid vector, the retroviral genes can then be inserted into the endogenous chromosomes of a mammalian host cell in one single step. Therefore, the use of a nucleic acid vector, as proposed herein, would reduce selection pressure, reduce the silencing timeframe and allow for faster screening of potential packaging cells. Furthermore, the retroviral genes included on the nucleic acid vector would all be integrated into the endogenous chromosomes of the mammalian host cell at a single locus which would reduce the risk of individual retroviral genes becoming silenced and ensure that all the retroviral genes are evenly expressed.

In one embodiment, the nucleic acid vector additionally comprises nucleic acid sequences which encode the RNA genome of a retroviral vector particle. It will be understood that the RNA genome of the retroviral vector particle is usually included on the "transfer vector" used in transient transfection methods. The transfer vector plasmid generally contains a promoter (such as CMV), the 3' LTR (which may or may not be a self-inactivating (*i.e*. SIN) 3'-LTR), the 5' LTR (which may or may not contain the U5 region), the encapsidation sequence (ψ) and potentially the transgene linked to a promoter.

In one embodiment, multiple copies of the RNA genome of the retroviral vector particle (*i.e*. the transfer vector) are included in the nucleic acid vector. Multiple copies of the transfer vector are expected to result in higher viral vector titre. For example, the nucleic acid vector may include two or more, such as three, four, five, six, seven, eight, nine or ten or more copies of the RNA genome of the retroviral vector particle (*i.e*. the transfer vector).

In one embodiment, the nucleic acid vector contains one or a plurality of recombination site(s). This allows for target sequences to be integrated into the endogenous chromosomes of the mammalian host cell in a site-specific manner in the presence of a recombinase enzyme. The recombinase enzyme catalyses the recombination reaction between two recombination sites.

Many types of site-specific recombination systems are known in the art, and any suitable recombination system may be used in the present invention. For example, in one embodiment the recombination site(s) are selected or derived from the *int*/*att* system of lambda phage, the Cre/lox system of bacteriophage P1, the FLP/FRT system of yeast, the Gin/gix recombinase system of phage Mu, the Cin recombinase system, the Pin recombinase system of *E. coli* and the R/RS system of the pSR1 plasmid, or any combination thereof. In a further embodiment, the recombination site is an *att* site (*e.g.* from lambda phage), wherein the *att* site permits site-directed integration in the presence of a lambda integrase. It will be understood that the reference to "lambda integrase" includes references to mutant integrases which are still compatible with the *int*/*att* system, for example the modified lambda integrases described in WO 2002/097059.

In one embodiment, the nucleic acid vector is selected from: a bacterial artificial chromosome (BAC), a yeast artificial chromosome (YAC), a P1-derived artificial chromosome (PAC), fosmid or a cosmid. In a further embodiment, the nucleic acid vector is a bacterial artificial chromosome (BAC).

### Bacterial artificial chromosomes

The term "bacterial artificial chromosome" or "BAC" refers to a DNA construct derived from bacterial plasmids which is able to hold a large insert of exogenous DNA. They can usually hold a maximum DNA insert of approximately 350 kb. BACs were developed from the well characterised bacterial functional fertility plasmid (F-plasmid) which contains partition genes that promote the even distribution of plasmids after bacterial cell division. This allows the BACs to be stably replicated and segregated alongside endogenous bacterial genomes (such as *E*. *coli*)*.* The BAC usually contains at least one copy of an origin of replication (such as the *oriS* or *oriV* gene), the *repE* gene (for plasmid replication and regulation of copy number) and partitioning genes (such as *sopA, sopB, parA, parB* and/or *parC*) which ensures stable maintenance of the BAC in bacterial cells. BACs are naturally circular and supercoiled which makes them easier to recover than linear artificial chromosomes, such as YACs. They can also be introduced into bacterial host cells relatively easily, using simple methods such as electroporation.

In one embodiment, the bacterial artificial chromosome comprises an *oriS* gene. In one embodiment, the bacterial artificial chromosome comprises a *repE* gene. In one embodiment, the bacterial artificial chromosome comprises partitioning genes. In a further embodiment, the partitioning genes are selected from *sopA, sopB, parA, parB* and/or *parC.* In a yet further embodiment, the bacterial artificial chromosome comprises a *sopA* and *sopB* gene.

BAC for use in the present invention may be obtained from commercial sources, for example the pSMART BAC from LUCIGEN^{™} (see Genome Accession No. EU101022.1 for the full back bone sequence). This BAC contains the L-arabinose "copy-up" system which also contains the *oriV* medium-copy origin of replication, which is active only in the presence of the TrfA replication protein. The gene for TrfA may be incorporated into the genome of bacterial host cells under control of the L-arabinose inducible promoter *araC-P_{BAD}* (see Wild etal. (2002) Genome Res. 12(9): 1434-1444). Addition of L-arabinose induces expression of TrfA, which activates *oriV,* causing the plasmid to replicate to up to 50 copies per cell.

### Yeast Artificial Chromosomes

The term "yeast artificial chromosome" or "YAC" refers to chromosomes in which yeast DNA is incorporated into bacterial plasmids. They contain an autonomous replication sequence (ARS) (*i.e*. an origin of replication), a centromere and telomeres. Unlike BACs, the YAC is linear and therefore contains yeast telomeres at each end of the chromosome to protect the ends from degradation as it is passed onto host cell progeny. YACs can hold a range of DNA insert sizes; anything from 100-2000 kb.

### P1-derived Artificial Chromosomes

The term "P1-derived artificial chromosome" or "PAC" refers to DNA constructs derived from the DNA of the P1-bacteriophage and bacterial F-plasmid. They can usually hold a maximum DNA insert of approximately 100-300 kb and are used as cloning vectors in *E. coli.* PACs have similar advantages as BACs, such as being easy to purify and introduce into bacterial host cells.

### Cosmids and Fosmids

The term "cosmid" refers to DNA constructs derived from bacterial plasmids which additionally contain *cos* sites derived from bacteriophage lambda. Cosmids generally contain a bacterial origin of replication (such as *oriV*), a selection marker, a cloning site and at least one *cos* site. Cosmids can usually accept a maximum DNA insert of 40-45 kb. Cosmids have been shown to be more efficient at infecting *E. coli* cells than standard bacterial plasmids. The term "fosmids" refers to non-mammalian nucleic acid vectors which are similar to cosmids, except that they are based on the bacterial F-plasmid. In particular, they use the F-plasmid origin of replication and partitioning mechanisms to allow cloning of large DNA fragments. Fosmids can usually accept a maximum DNA insert of 40 kb.

### RETROVIRUSES

Retroviruses are a family of viruses which contain a pseudo-diploid single-stranded RNA genome. They encode a reverse transcriptase which produces DNA from the RNA genome which can then be inserted into the host cell DNA. The invention described herein may be used to produce replication defective retroviral vector particles. The retroviral vector particle of the present invention may be selected from or derived from any suitable retrovirus.

In one embodiment, the retroviral vector particle is derived from, or selected from, a lentivirus, alpha-retrovirus, gamma-retrovirus or foamy-retrovirus, such as a lentivirus or gamma-retrovirus, in particular a lentivirus. In a further embodiment, the retroviral vector particle is a lentivirus selected from the group consisting of HIV-1, HIV-2, SIV, FIV, EIAV and Visna. Lentiviruses are able to infect non-dividing (*i.e*. quiescent) cells which makes them attractive retroviral vectors for gene therapy. In a yet further embodiment, the retroviral vector particle is HIV-1 or is derived from HIV-1. The genomic structure of some retroviruses may be found in the art. For example, details on HIV-1 may be found from the NCBI Genbank (Genome Accession No. AF033819). HIV-1 is one of the best understood retroviruses and is therefore often used as a retroviral vector.

### Retroviral Genes

The nucleic acid sequences common to all retroviruses may be explained in more detail, as follows:
Long Terminal Repeats (LTRs): The basic structure of a retrovirus genome comprises a 5'-LTR and a 3'-LTR, between or within which are located the genes required for retroviral production. The LTRs are required for retroviral integration and transcription. They can also act as promoter sequences to control the expression of the retroviral genes (*i.e*. they are *cis*-acting genes). The LTRs are composed of three sub-regions designated U3, R, U5: U3 is derived from the sequence unique to the 3' end of the RNA; R is derived from a sequence repeated at both ends of the RNA; and U5 is derived from the sequence unique to the 5' end of the RNA. Therefore, in one embodiment, the nucleic acid vector additionally comprises a 5'- and 3'-LTR. In a further embodiment, the U5 region of the 5' LTR can be deleted and replaced with a non-HIV-1 polyA tail (see Hanawa et al. (2002) Mol. Ther. 5(3): 242-51).

In order to address safety concerns relating to the generation of replication-competent virus, a self-inactivating (SIN) vector has been developed by deleting a section in the U3 region of the 3' LTR, which includes the TATA box and binding sites for transcription factors Sp1 and NF-κB (see Miyoshi et al. (1998) J. Virol. 72(10):8150-7). The deletion is transferred to the 5' LTR after reverse transcription and integration in infected cells, which results in the transcriptional inactivation of the LTR. This is known as a self-inactivating lentiviral-based vector system which may be included in the present invention.

ψ: Encapsidation of the retroviral RNAs occurs by virtue of a ψ (psi) sequence located at the 5' end of the retroviral genome. It is also well known in the art that sequences downstream of the psi sequence and extending into the *gag* coding region are involved in efficient retroviral vector production (see Cui etal. (1999) J. Virol. 73(7): 6171-6176). In one embodiment, the nucleic acid vector additionally comprises a ψ (psi) sequence.

Primer Binding Site (PBS): The retroviral genome contains a PBS which is present after the U5 region of the 5'-LTR. This site binds to the tRNA primer required for initiation of reverse transcription. In one embodiment, the nucleic acid vector additionally comprises a PBS sequence.

PPT: Retroviral genomes contain short stretches of purines, called polypurine tracts (PPTs), near the 3' end of the retroviral genome. These PPTs function as RNA primers for plus-strand DNA synthesis during reverse transcription. Complex retroviruses (such as HIV-1) contain a second, more centrally located PPT (*i.e*. a central polypurine tract (cPPT)) that provides a second site for initiation of DNA synthesis. Retroviral vectors encoding a cPPT have been shown to have enhanced transduction and transgene expression (see Barry etal. (2001) Hum. Gene Ther. 12(9):1103-8). In one embodiment, the nucleic acid vector additionally comprises a 3'-PPT sequence and/or a cPPT sequence.

The genomic structure of the non-coding regions described above are well known to a person skilled in the art. For example, details on the genomic structure of the non-coding regions in HIV-1 may be found from the NCBI Genbank with Genome Accession No. AF033819, or for HIV-1 HXB2 (a commonly used HIV-1 reference strain) with Genome Accession No. K03455. In one embodiment, the non-coding regions are derived from the sequences available at Genome Accession No. K03455, for example from base pairs 454-1126 (for R-U5-PBS-Gag), 7622-8479 (for RRE) or 7769-8146 (for RRE), 4781-4898 (for cPPT), 9015-9120 & 9521-9719 (for dNEF-PPT-sinU3-R-U5).

*Gag*/*pol*: The expression of *gag* and *pol* genes relies on a translational frameshift between *gag* and *gagpol.* Both are polyproteins which are cleaved during maturation. The major structural matrix, capsid, and nucleocapsid proteins of the retroviral vector are encoded by *gag.* The *pol* gene codes for the retroviral enzymes: i) reverse transcriptase, essential for reverse transcription of the retroviral RNA genome to double stranded DNA, ii) integrase, which enables the integration of the retroviral DNA genome into a host cell chromosome, and iii) protease, that cleaves the synthesized polyprotein in order to produce the mature and functional proteins of the retrovirus. In one embodiment, the retroviral nucleic acid sequence encoding the gag and pol proteins is derived from the HIV-1 HXB2 sequence, which is available at Genome Accession No. K03455, for example from base pairs 790-5105.

*Env*: The *env* ("envelope") gene codes for the surface and transmembrane components of the retroviral envelope (*e.g*. glycoproteins gp120 and gp41 of HIV-1) and is involved in retroviral-cell membrane fusion. In order to broaden the retroviral vector's tissue tropism, the retroviral vectors described herein may be pseudotyped with an envelope protein from another virus. Pseudotyping refers to the process whereby the host cell range of retroviral vectors, including lentiviral vectors, can be expanded or altered by changing the glycoproteins (GPs) on the retroviral vector particles (*e.g*. by using GPs obtained from or derived from other enveloped viruses or using synthetic/artificial GPs). The most commonly used glycoprotein for pseudotyping retroviral vectors is the Vesicular stomatitis virus GP (VSVg), due to its broad tropism and high vector particle stability. However, it will be understood by the skilled person that other glycoproteins may be used for pseudotyping (see Cronin et al. (2005) Curr. Gene Ther. 5(4):387-398,). The choice of virus used for pseudotyping may also depend on the type of cell and/or organ to be targeted because some pseudotypes have been shown to have tissue-type preferences.

In one embodiment, the env protein or a functional substitute thereof is obtained from or derived from a virus selected from a Vesiculovirus (*e.g*. Vesicular stomatitis virus), Lyssavirus (*e.g*. Rabies virus, Mokola virus), Arenavirus (*e.g*. Lymphocytic choriomeningitis virus (LCMV)), Alphavirus (*e.g*. Ross River virus (RRV), Sindbis virus, Semliki Forest virus (SFV), Venezuelan equine encephalitis virus), Filovirus (*e.g*. Ebola virus Reston, Ebola virus Zaire, Lassa virus), Coronavirus (*e.g*. SARS-CoV), Respirovirus (*e.g*. Sendai virus, Respiratory syncytia virus (RSV)), Hepacivirus (*e.g*. Hepatitis C virus (HCV)), Influenzavirus (*e.g*. Influenza virus A) and Nucleopolyhedrovirus (*e.g.* Autographa californica multiple nucleopolyhedrovirus (AcMNPV)). In a further embodiment, the env protein or a functional substitute thereof is obtained from or derived from Vesicular stomatitis virus. In this embodiment, he Vesicular stomatitis virus glycoprotein (VSVg) protein may be used which enables the retroviral particles to infect a broader host cell range and eliminates the chances of recombination to produce wild-type envelope proteins. In a further embodiment, the nucleic acid sequence encoding the env protein, is derived from the sequence available at Genome Accession No. J02428.1, for example from base pairs 3071 to 4720.

The structural genes described herein are common to all retroviruses. Further auxiliary genes may be found in different types of retrovirus. For example, lentiviruses, such as HIV-1, contain six further auxiliary genes known as *rev, vif, vpu, vpr, nef* and *tat.* Other retroviruses may have auxiliary genes which are analogous to the genes described herein, however they may not have always been given the same name as in the literature. References such as Tomonaga and Mikami (1996) J. Gen. Virol. 77(Pt 8):1611-1621 describe various retrovirus auxiliary genes.

*Rev*: The auxiliary gene *rev* ("regulator of virion") encodes an accessory protein which binds to the Rev Response element (RRE) and facilitates the export of retroviral transcripts. The gene's protein product allows fragments of retroviral mRNA that contain the Rev Responsive element (RRE) to be exported from the nucleus to the cytoplasm. The RRE sequence is predicted to form a complex folded structure. This particular role of rev reflects a tight coupling of the splicing and nuclear export steps. In one embodiment, nucleic acid vector comprises an RRE sequence. In a further embodiment, the RRE sequence is derived from HIV-1 HXB2 sequence, which is available at Genome Accession No. K03455, for example from base pairs 7622 to 8479, or 7769 to 8146, in particular base pairs 7622 to 8479.

Rev binds to RRE and facilitates the export of singly spliced (*env, vif, vpr* and *vpu*) or non-spliced (*gag, pol* and genomic RNA) viral transcripts, thus leading to downstream events like gene translation and packaging (see Suhasini and Reddy (2009) Curr. HIV Res. 7(1): 91-100). In one embodiment, the nucleic acid vector additionally comprises the auxiliary gene *rev* or an analogous gene thereto (*i.e.* from other retroviruses or a functionally analogous system). Inclusion of the *rev* gene ensures efficient export of RNA transcripts of the retroviral vector genome from the nucleus to the cytoplasm, especially if an RRE element is also included on the transcript to be transported. In a further embodiment, the *rev* gene comprises at least 60% sequence identity, such as at least 70% sequence identity to base pairs 970 to 1320 of Genome Accession No. M11840 (*i.e*. HIV-1 clone 12 cDNA, the HIVPCV12 locus). In an alternative embodiment, the *rev* gene comprises at least 60% sequence identity, such as at least 70%, 80%, 90% or 100% sequence identity to base pairs 5970 to 6040 and 8379 to 8653 of Genome Accession No. K03455.1 (*i.e*. Human immunodeficiency virus type 1, HXB2).

Auxiliary genes are thought to play a role in retroviral replication and pathogenesis, therefore many current viral vector production systems do not include some of these genes. The exception is *rev* which is usually present or a system analogous to the *rev*/RRE system is potentially used. Therefore, in one embodiment, the nucleic acid sequences encoding one or more of the auxiliary genes *vpr, vif, vpu, tat* and *nef,* or analogous auxiliary genes, are disrupted such that said auxiliary genes are removed from the RNA genome of the retroviral vector particle or are incapable of encoding functional auxiliary proteins. In a further embodiment, at least two or more, three or more, four or more, or all of the auxiliary genes *vpr, vif, vpu, tat* and *nef,* or analogous auxiliary genes, are disrupted such that said auxiliary genes are removed from the RNA genome of the retroviral vector particle or are incapable of encoding functional auxiliary proteins. Removal of the functional auxiliary gene may not require removal of the whole gene; removal of a part of the gene or disruption of the gene will be sufficient.

It will be understood that the nucleic acid sequences encoding the replication defective retroviral vector particle may be the same as, or derived from, the wild-type genes of the retrovirus upon which the retroviral vector particle is based, i.e. the sequences may be genetically or otherwise altered versions of sequences contained in the wild-type virus. Therefore, the retroviral genes incorporated into the nucleic acid vectors or host cell genomes, may also refer to codon-optimised versions of the wild-type genes.

### ADDITIONAL COMPONENTS

The nucleic acid vectors of the invention may comprise further additional components. These additional features may be used, for example, to help stabilize transcripts for translation, increase the level of gene expression, and turn on/off gene transcription.

The retroviral vector particles produced by the invention may be used in methods of gene therapy. Therefore, in one embodiment, the nucleic acid vector additionally comprises one or more transgenes. This transgene may be a therapeutically active gene which encodes a gene product which may be used to treat or ameliorate a target disease. The transgene may encode, for example, an antisense RNA, a ribozyme, a protein (for example a tumour suppressor protein), a toxin, an antigen (which may be used to induce antibodies or helper T-cells or cytotoxic T-cells) or an antibody (such as a single chain antibody). In one embodiment, the transgene encodes beta globin.

Multiple copies of the transfer vector containing the transgene are expected to result in higher retroviral vector titre, therefore in one embodiment, the nucleic acid vector comprises multiple copies of the transgene, such as two or more, in particular three or more, copies of the transgene. In some cases more than one gene product is required to treat a disease, therefore in a further embodiment, the nucleic acid vector additionally comprises two or more, such as three or more, or four or more, different transgenes.

References herein to "transgene" refer to heterologous or foreign DNA which is not present or not sufficiently expressed in the mammalian host cell in which it is introduced. This may include, for example, when a target gene is not expressed correctly in the mammalian host cell, therefore a corrected version of the target gene is introduced as the transgene. Therefore, the transgene may be a gene of potential therapeutic interest. The transgene may have been obtained from another cell type, or another species, or prepared synthetically. Alternatively, the transgene may have been obtained from the host cell, but operably linked to regulatory regions which are different to those present in the native gene. Alternatively, the transgene may be a different allele or variant of a gene present in the host cell.

The aim of gene therapy is to modify the genetic material of living cells for therapeutic purposes, and it involves the insertion of a functional gene into a cell to achieve a therapeutic effect. The retroviral vector produced using the nucleic acid vectors and host cells described herein can be used to transfect target cells and induce the expression of the gene of potential therapeutic interest. The retroviral vector can therefore be used for treatment of a mammalian subject, such as a human subject, suffering from a condition including but not limited to, inherited disorders, cancer, and certain viral infections.

In one embodiment, the nucleic acid vector additionally comprises a transcription regulation element. For example, any of the elements described herein may be operably linked to a promoter so that expression can be controlled. Promoters referred to herein may include known promoters, in whole or in part, which may be constitutively acting or inducible, *e.g.* in the presence of a regulatory protein. In one embodiment, the nucleic acid vector additionally comprises a high efficiency promoter, such as a CMV promoter. This promoter has the advantage of promoting a high level of expression of the elements encoded on the non-mammalian nucleic acid vector. In a further embodiment, the CMV promoter comprises a sequence derived from the human cytomegalovirus strain AD169. This sequence is available at Genome Accession No. X17403, for example from base pairs 173731 to 174404.

In one embodiment, the promoter (such as a CMV promoter) additionally comprises at least one Tet operon. The Tet operon system may be used to control expression of the retroviral sequences contained within the nucleic acid vector. Briefly, the Tet repressor protein blocks expression by binding to the Tet operon site which is introduced into the promoter. Therefore, when the Tet repressor is bound to the Tet operon, there is no gene expression. On addition of tetracycline or doxycyclin, the Tet repressor is sequestered allowing promoter activity, therefore gene expression is switched on. Tet operon systems are widely available, such as the Tet operon used in the pcDNN^{™}4/TO mammalian expression vector available from Invitrogen.

In one embodiment, the nucleic acid vector additionally comprises a tetracycline resistance operon repressor protein ("Tet repressor" or "TetR"). In a further embodiment, the Tet repressor is codon optimised.

In one embodiment, the nucleic acid vector additionally comprises an insulator, such as a chromatin insulator. The term "insulator" refers to a genetic sequence which blocks the interaction between promoters and enhancers. In a further embodiment, the insulator (such as a chromatin insulator) is present between each of the retroviral nucleic acid sequences. This helps to prevent promoter interference (*i.e*. where the promoter from one transcription unit impairs expression of an adjacent transcription unit) between adjacent retroviral nucleic acid sequences. It will be understood that if the insulators are present in the nucleic acid vector between each of the retroviral nucleic acid sequences, then these may be arranged as individual expression constructs within the nucleic acid vector. For example, each sequence encoding the retroviral nucleic acid sequences has its own promoter and/or an intron and/or polyA signal. In one embodiment, the chromatin insulator has at least 90% sequence identity, for example at least 95% sequence identity, to the chicken (*Gallus gallus*) HS4 insulator sequence (for example see Genome Accession No. U78775.2, base pairs 1 to 1205).

In one embodiment, the nucleic acid vector additionally comprises a selectable marker. This allows the cells which have incorporated the nucleic acid sequences encoding a replication defective retroviral vector particle to be selected. In a further embodiment, the selectable marker is an antibiotic resistance gene, such as a zeocin, kanamycin or puromycin resistance gene, in particular a zeocin (ZeoR) resistance gene. In a yet further embodiment, the zeocin resistance gene is derived from the *Streptoalloteichus hindustans ble* gene, for example see Genome Accession No. X52869.1 from base pairs 3 to 377.

In one embodiment, the nucleic acid vector additionally comprises a polyA signal. The use of a polyA signal has the advantage of protecting mRNA from enzymatic degradation and aiding in translation. In a further embodiment, the polyA signal is obtained from or derived from SV40, Bovine Growth Hormone and/or Human Beta Globin. In one embodiment, the polyA signal is derived from the SV40 early polyA signal (for example, see Genome Accession No. EF579804.1, base pairs 2668 to 2538 from the minus strand). In one embodiment, the polyA signal is derived from the Human Beta Globin polyA signal (for example, see Genome Accession No. GU324922.1, base pairs 3394 to 4162).

In one embodiment, the nucleic acid vector additionally comprises an intron sequence. The use of an intron downstream of the enhancer/promoter region and upstream of the cDNA insert (*i.e*. the transgene) is known to increase the level of expression of the insert. In a further embodiment, the intron sequence is a Human Beta Globin Intron or the Rabbit Beta Globin Intron II sequence. In one embodiment, the Human Beta Globin Intron is derived from the sequence available at Genome Accession No. KM504957.1 (for example from base pairs 476 to 1393). In one embodiment, the Rabbit Beta Globin Intron II is derived from the sequence available at Genome Accession No. V00882.1 (for example, from base pairs 718 to 1290).

In one embodiment, the nucleic acid vector additionally comprises a woodchuck hepatitis virus post-transcriptional regulatory element (WPRE). The presence of WPRE has been shown to enhance expression and as such is likely to be beneficial in attaining high levels of expression. In a further embodiment, the WPRE is derived from the sequence available at Genome Accession No. J04514.1 (for example, from base pairs 1093 to 1684).

In one embodiment, the nucleic acid vector additionally comprises an internal ribosome entry site (IRES). An IRES is a structured RNA element that is usually found in the 5'-untranslated region downstream of the 5'-cap (which is required for the assembly of the initiation complex). The IRES is recognized by translation initiation factors, and allows for cap-independent translation. In a further embodiment, the IRES is derived from the Encephalomyocarditis virus (EMCV) genome (for example, see Genome Accession No. KF836387.1, base pairs 151 to 724).

In one embodiment, the nucleic acid vector additionally comprises a Multiple Cloning Site (MCS). An MCS is a short segment of DNA within the nucleic acid vector which contains multiple restriction sites (for example, 10, 15 or 20 sites). These sites usually occur only once within the nucleic acid vector to ensure that the endonuclease only cuts at one site. This allows for the retroviral genes to be easily inserted using the appropriate endonucleases (*i.e*. restriction enzymes).

It will be understood by a person skilled in the art that the constructs may be arranged in any order within the nucleic acid vector. In an exemplary embodiment, the nucleic acid vector comprises the following insert: a retroviral nucleic acid sequence encoding the gag and pol proteins, a VSVg nucleic acid sequence encoding the env protein., a retroviral nucleic acid sequence encoding the auxiliary gene *rev* (such as a codon optimised *rev* sequence) or an analogous gene thereto or a functionally analogous system, a tetracycline resistance operon repressor protein (TetR), an internal ribosome entry site, and a selectable marker (such as a zeocin resistance selection marker) (*i.e*., GagPol-Env-Rev-TetRepressor-IRES-Antibiotic Resistance marker-remaining BAC sequence ("BAC bone"); such as: GagPol-(wild-type)VSVg-(codon-optimised)Rev-TetRepressor-IRES-ZeocinResistance-pSMARTBAC). In a further embodiment, an insulator (such as a chromatin insulator) is present between each of the *gagpol, env* and rev sequences. In a further embodiment, a promoter is present before each of the *gagpol, env* and rev sequences. In a yet further embodiment, at least one copy of the transfer vector sequence (*i.e*. comprising nucleic acid sequences which encode the RNA genome of a retroviral vector particle) is present before the *gagpol* sequence.

In one embodiment, the nucleic acid vector comprises the following insert: an insulator (such as a chromatin insulator), a promoter (such as a CMV promoter optionally comprising a Tet operon sequence), an intron (such as a human beta globin intron), a retroviral nucleic acid sequence encoding the gag and pol proteins, a retroviral nucleic acid encoding RRE, a polyA signal (such as a human beta globin polyA signal), an insulator (such as a chromatin insulator), a promoter (such as a CMV promoter optionally comprising a Tet operon sequence), an intron (such as a human beta globin intron), a VsVg nucleic acid sequence encoding the env protein, a polyA signal (such as a human beta globin polyA signal), an insulator (such as a chromatin insulator), a promoter (such as a CMV promoter optionally comprising a Tet operon sequence), a retroviral nucleic acid sequence encoding the auxiliary gene *rev* or an analogous gene thereto or a functionally analogous system, a polyA signal (such as a human beta globin polyA signal), an insulator (such as a chromatin insulator), a promoter (such as a CMV promoter), an intron (such as a rabbit beta globin intron), a tetracycline resistance operon repressor protein (TetR), an internal ribosome entry site, a selectable marker (such as a zeocin resistance selection marker), a polyA signal and a multiple cloning site.

The nucleic acid sequences may be introduced into the nucleic acid vector sequentially. This allows for selection after each integration to ensure that all of the required nucleic acid sequences are successfully integrated into the nucleic acid vector. Alternatively, at least two or more of the nucleic acid sequences are introduced into the nucleic acid vector simultaneously.

It will be understood that the additional genes described herein may be introduced into the nucleic acid vector by standard molecular cloning techniques known in the art, for example using restriction endonucleases and ligation techniques. Furthermore, the nucleic acid vector, in particular BACs, PACs, fosmids and/or cosmids, may be introduced into bacterial host cells (such as *E. coli* cells, in particular the *E. coli* strain DH10B) by standard techniques, such as electroporation.

### USES

According to a further aspect of the invention, there is provided the nucleic acid vector as defined herein for use in producing a retroviral packaging or producer cell line.

The nucleic acid vectors described herein may be used to create a retroviral packaging cell line which would greatly simplify retroviral vector production. It will be understood that if a transgene is included on the nucleic acid vector, then this would be used to create a producer cell line.

As described herein, it would be useful to develop a stable retroviral packaging (or producer) cell line in order to overcome the difficulties associated with transient transfection. The nucleic acid vectors described herein can be used to prepare said packaging cell lines because they are able to hold large DNA inserts containing the essential genes required for retroviral packaging which can then be integrated into the endogenous genome of mammalian host cells in one step.

### HOST CELLS

This specification describes a retroviral packaging cell comprising nucleic acid sequences encoding:
gag and pol proteins; and
env protein or a functional substitute thereof, wherein said nucleic acid sequences are all located at a single locus within the retroviral packaging cell genome.

The advantage of including all of the retroviral genes on a large nucleic acid vector is that they can be prepared in microbial cells (such as bacterial or yeast cells) first, which are much easier to handle and manipulate, before being integrated into mammalian cells in a single step. This relieves selection pressure and reduces the silencing timeframe once the retroviral genes have been integrated into a mammalian host cell. The characteristic feature of this method is that all of the retroviral genes required to create a packaging cell line are present in a single locus in the endogenous genome, rather than randomly scattered throughout the endogenous genome. This has the advantage of producing a retroviral packaging cell which expresses all of the retroviral genes at the same level because they are located at the same locus, as compared to previous methods where the retroviral genes are integrated randomly throughout the endogenous genome which may cause uneven levels of expression.

In one embodiment, the retroviral packaging cell additionally comprises nucleic acid sequences which encode the RNA genome of the retroviral vector particle. This may also be located at the single locus with the nucleic acid sequences encoding the gag and pol proteins and the env protein or a functional substitute thereof.

This specification describes a retroviral producer cell comprising nucleic acid sequences encoding:
gag and pol proteins;
env protein or a functional substitute thereof; and
the RNA genome of the retroviral vector particle,
wherein said nucleic acid sequences are all located at a single locus within the retroviral producer cell genome.

In one embodiment, the retroviral packaging cell is a mammalian cell. In a further embodiment, the mammalian cell is selected from a HEK 293 cell, CHO cell, Jurkat cell, KS62 cell, PerC6 cell, HeLa cell or a derivative or functional equivalent thereof. In a yet further embodiment, the mammalian host cell is a HEK 293 cell, or derived from a HEK 293 cell. Such cells could be adherent cell lines (*i.e*. they grow in a single layer attached to a surface) or suspension adapted/non-adherent cell lines (*i.e*. they grow in suspension in a culture medium). In a yet further embodiment, the HEK 293 cell is a HEK 293T cell. The term "HEK 293 cell" refers to the Human Embryonic Kidney 293 cell line which is commonly used in biotechnology. In particular, HEK 293T cells are commonly used for the production of various retroviral vectors. Other examples of suitable commercially available cell lines include T-REX^{™} (Life Technologies) cell lines.

### METHODS

According to a further aspect of the invention, there is provided a method of producing a stable retroviral packaging cell line, comprising:
(a) introducing a nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid. comprising retroviral nucleic acid sequences encoding:
   gag and pol proteins, and
   an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus
   into a culture of mammalian host cells; and
(b) selecting within the culture for a mammalian host cell which has the nucleic acid sequences encoded on the vector integrated into an endogenous chromosome of the mammalian host cell.

In one embodiment, the mammalian host cell is selected from a HEK 293 cell, HEK 6E cell, CHO cell, Jurkat cell, KS62 cell, PerC6 cell, HeLa cell or a derivative or functional equivalent thereof. In a further embodiment, the mammalian host cell is a HEK 293 cell, or derived from a HEK 293 cell. Such cells could be adherent cell lines (*i.e*. they grow in a single layer attached to a surface) or suspension adapted/non-adherent cell lines (*i.e*. they grow in suspension in a culture medium). In a yet further embodiment, the HEK 293 cell is a HEK 293T cell or HEK 6E cell. Other examples of suitable commercially available cell lines include T-REX^{™} (Life Technologies) cell lines.

The skilled person will be aware that introducing the nucleic acid vector into the host cell may be performed using suitable methods known in the art, for example, lipid-mediated transfection, microinjection, cell (such as microcell) fusion, electroporation or microprojectile bombardment. In one embodiment, the nucleic acid vector is introduced into the host cell by electroporation. It will be understood that the choice of method to use for introducing the nucleic acid vector can be chosen depending upon the type of mammalian host cell used.

Once inside the mammalian host cell, the nucleic acid vector will randomly integrate into the endogenous genome of the mammalian host cell. Therefore, the method additionally comprises selecting for the mammalian host cell in which the nucleic acids encoded on the nucleic acid vector have integrated (for example, using an antibiotic resistance selection marker, such as a zeocin resistance marker).

The skilled person will be aware of methods to encourage integration of the nucleic acid vector, for example, linearising the nucleic acid vector if it is naturally circular (for example, BACs, PACs, cosmids or fosmids). The nucleic acid vector may additionally comprise areas of shared homology with the endogenous chromosomes of the mammalian host cell to guide integration to a selected site within the endogenous genome. Furthermore, if recombination sites are present on the nucleic acid vector then these can be used for targeted recombination. For example, the nucleic acid vector may contain a *IoxP* site which allows for targeted integration when combined with Cre recombinase (*i.e.* using the Cre/lox system derived from P1 bacteriophage). Alternatively (or additionally), the recombination site is an att site (*e.g.* from lambda phage), wherein the *att* site permits site-directed integration in the presence of a lambda integrase. This would allow the retroviral genes to be targeted to a locus within the endogenous genome which allows for high and/or stable expression.

Other methods of targeted integration are well known in the art. For example, methods of inducing targeted cleavage of genomic DNA can be used to encourage targeted recombination at a selected chromosomal locus. These methods often involve the use of engineered cleavage systems to induce a double strand break (DSB) or a nick in the endogenous genome to induce repair of the break by natural processes such as non-homologous end joining (NHEJ) or repair using a repair template (*i.e*., homology directed repair or HDR).

Cleavage can occur through the use of specific nucleases such as engineered zinc finger nucleases (ZFN), transcription-activator like effector nucleases (TALENs), using the CRISPR/Cas9 system with an engineered crRNA/tracr RNA ('single guide RNA') to guide specific cleavage, and/or using nucleases based on the Argonaute system (*e.g.,* from *T. thermophilus,* known as 'TtAgo', see Swarts et al. (2014) Nature 507(7491): 258-261). Targeted cleavage using one of these nuclease systems can be exploited to insert a nucleic acid into a specific target location using either HDR or NHEJ-mediated processes. Therefore, in one embodiment, the method additionally comprises integrating the nucleic acid sequences encoded on the nucleic acid vector into the genome (*i.e*. an endogenous chromosome) of the mammalian host cell using at least one nuclease, wherein the at least one nuclease cleaves the genome of the mammalian host cell such that the nucleic acid sequences are integrated into the genome of the cell. In a further embodiment, the at least one nuclease is selected from the group consisting of a zinc finger nuclease (ZFN), a TALE nuclease (TALEN), a CRISPR/Cas nuclease system and combinations thereof.

According to a further aspect of the invention, there is provided a retroviral packaging cell obtained by the method defined herein.

The cell line obtained using the methods defined herein may be used to produce a high titre of retroviral vector.

References herein to the term "high titre" refer to an effective amount of retroviral vector or particle which is capable of transducing a target cell, such as a patient cell. In one embodiment, a high titre is in excess of 10⁶ TU/ml without concentration (TU = transducing units).

According to a further aspect of the invention, there is provided a method of producing a replication defective retroviral vector particle, comprising:
(a) introducing a nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid. comprising retroviral nucleic acid sequences encoding:
   gag and pol proteins, and
   an env protein obtained from or derived form a virus selected from a Vesiculovirus, Lyssavirus, Arenavirus, Alphavirus, Filovirus, Coronavirus, Respirovirus, Hepacivirus, Influenzavirus and Nucleopolyhedrovirus
   wherein each of the retroviral nucleic acid sequences has its own promoter within the nucleic acid vector into a culture of mammalian host cells into a culture of mammalian host cells;
(b) selecting within the culture for a mammalian host cell which has the nucleic acid sequences encoded on the vector integrated into an endogenous chromosome of the mammalian host cell; and
(c) further culturing the mammalian host cell under conditions in which the replication defective retroviral vector particle is produced.

As described hereinbefore, in one embodiment, the mammalian host cell is selected from a HEK 293 cell, CHO cell, Jurkat cell, KS62 cell, PerC6 cell, HeLa cell or a derivative or functional equivalent thereof. In a further embodiment, the mammalian host cell is a HEK 293 cell, or derived from a HEK 293 cell. Such cells could be adherent cell lines (*i.e*. they grow in a single layer attached to a surface) or suspension adapted/non-adherent cell lines (*i.e*. they grow in suspension in a culture medium). In a yet further embodiment, the HEK 293 cell is a HEK 293T cell. Other examples of suitable commercially available cell lines include T REX^{™} (Life Technologies) cell lines.

It will be understood by the skilled person that the conditions used in the method described herein will be dependent upon the host cell used. Typical conditions, for example the culture medium or temperature to be used, are well known in the art. In one embodiment, culturing is performed by incubating the mammalian host cell under humidified conditions. In a further embodiment, the humidified conditions comprise incubating the transfected cells at 37°C at 5% CO₂. In one embodiment, culturing is performed using a culture medium selected from: Dulbecco's modified Eagle's medium (DMEM) containing 10% (vol/vol) fetal bovine serum (FBS), serum-free UltraCULTURE^{™} medium (Lonza, Cat. No. 12-725F), or Freestyle^{™} Expression medium (Thermo fisher, Cat. No. 12338-018).

In one embodiment, the method additionally comprises isolating the replication defective retroviral vector particle. For example, in one embodiment the isolating is performed by using a filter. In a further embodiment, the filter is a low-protein binding membrane (*e.g*. a 0.22µm low-protein binding membrane or a 0.45µm low-protein binding membrane), such as polyvinylidene fluoride (PVDF) or polyethersulfone (PES) artificial membranes.

Once inside the mammalian host cell, the retroviral nucleic acids present on the nucleic acid vector may integrate into a random, single locus within the endogenous genome. The integration step may be encouraged as described hereinbefore, for example using linearisation and/or areas of shared homology. Recombination sites may also be used for targeted recombination.

If the target genes are integrated into the endogenous chromosomes with a selective marker, such as an antibiotic resistance gene, then the method may additionally comprise selecting for the mammalian host cells in which the retroviral nucleic acids have successfully integrated.

Once isolated, the retroviral vector particles may be concentrated for *in vivo* applications. Concentration methods include, for example, ultracentrifugation, precipitation or anion exchange chromatography. Ultracentrifugation is useful as a rapid method for retroviral vector concentration at a small scale. Alternatively, anion exchange chromatography (for example using Mustang Q anion exchange membrane cartridges) or precipitation (for example using PEG 6000) are particularly useful for processing large volumes of lentiviral vector supernatants.

The invention will now be described in further detail with reference to the following, nonlimiting Examples.

### EXAMPLES

### EXAMPLE 1: Construct Guide

**Figure 1** shows a stepwise guide to the construction of BACpack-WTGP-277deIU5 and BACpack-SYNGP-277deIU5. Owing to the compatible ends of an XbaI and NheI digest, the lentiviral packaging genes were progressively loaded into the pSmart BAC vector. At the point of GagPol addition, 2 constructs were made containing either Wild type GagPol (WTGP) or the codon optimised GagPol, SYNGP. These were given the nomenclature of BACpack-WTGP and of BACpack-SYNGP respectively. The transfer cassette was then loaded onto both of these constructs and so generating BACpackWTGP-277deIU5 and BACpackSYNGP-277deIU5.

### EXAMPLE 2: Selection of a stable polyclonal pool

Polyclonal stable transfectant pools were generated by transfecting the adherent cell line, HEK293T, with BACpackSYNGP-277deIU5 or BACpackWTGP-277deIU5. Successful integration events were then selected for with Zeocin.

To assess the ability of these polyclonal pools to generate lentiviral vector, the cells were induced with Doxycycline (I) or left un-induced (UI) and compared to untransfected HEK293T cells.

The results show the titre in transduction units (TU)/mL, of the lentiviral vector supernatant harvested from each transfection condition. It can be seen from the titration results in **Figure 2** that the stable polyclonal pools, generated with either BACpackSYNGP-277deIU5 or BACpackWTGP-277deIU5 are capable of producing lentiviral vector at concentrations in region of 10e7 TU/mL which is comparable to the current transient transfection system.

The results confirm that the single BAC vector containing all of the packaging genes necessary for lentiviral production can generate cell lines capable of producing lentiviral vector at suitable titre.

### EXAMPLE 3: Generating stable transfection suspension clones

The primary purpose of generating lentiviral vector producing cell lines using the BAC technology is to rapidly apply new advances to the platform. These advances are likely to include modification of specialist cell lines. For example, it is an industry standard to increase yield by producing biological products in suspension cells as they grow to greater densities than adherent cells. However, the current lentiviral vector production system relies on high transfection rates which are harder to achieve in suspension cells than adherent HEK293T cells. As transfection efficiency is less of a concern when generating a stable cell line due to the selection of successful integrants, the BAC construct is an ideal solution to generate lentiviral vector producing suspension cell lines.

As previously demonstrated, the BAC construct is capable of generating lentiviral vector producer cell lines from adherent HEK293T cells. To prove the flexibility of the BAC constructs, stable transfectant cell lines were generated from the suspension cell line HEK293 6E. The HEK293 6E cells were transfected with the BAC construct BACpackWTGP-277deIU5 then selected with Zeocin. This was followed by cloning to generate clonal cell lines. The results in **Figure 3** show the GFP signal generated by the stable cell lines. This indicates both the presence of Zeocin resistance and a functional GFP expression cassette in the transfer vector segment.

This result suggests that the BAC construct is capable of generating stable clones from multiple cell lines.

### EXAMPLE 4: Induction of Lentivirus in the suspension clones

In order to confirm the ability of the stable suspension clones to produce lentiviral vector, clones 1, 14, 15 and 16, were induced with 2µg/ml doxycycline and the supernatant measured for viral titre by transduction of HEK293T cells.

The results in **Figure 4** show the titre in transduction units (TU)/mL, of the lentiviral vector supernatant harvested from each clone. The results clearly show that cell lines generated by stable transfection of the suspension cell line HEK293 6E with BACpackWTGP-277deIU5 are capable of producing lentiviral vector at yields comparable to the current transient transfection system.

### EXAMPLE 5: Vector titre of clones

Clones 1 and 16 as described in **Figure 4** were passaged in culture and induced and titred at later timepoints to determine whether vector production was stable from these highly productive clones. As shown in **Figures 5A** and **5B****,** vector titres from these clones actually increased modestly between passage 5 and passage 21, possibly due to an increase in sodium butyrate concentration introduced into the induction method.

It will be understood that the embodiments described herein may be applied to all aspects of the invention.

## Claims

1. A method of producing a stable retroviral packaging cell line, comprising:
(a) introducing a nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid comprising retroviral nucleic acid sequences encoding:
gag and pol proteins, and
an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus,
wherein each of the nucleic acid sequences has its own promoter within the nucleic acid vector, into a culture of mammalian host cells; and
(b) selecting within the culture for a mammalian host cell which has the nucleic acid sequences encoded on the vector integrated into an endogenous chromosome of the mammalian host cell.

2. A method according to claim 1, wherein the nucleic acid vector additionally comprises nucleic acid sequences which encode the RNA genome of a retroviral vector particle.

3. A method according to claims 1 or 2, wherein the nucleic acid vector additionally comprises the auxiliary gene rev.

4. A method according to any one of claims 1 to 3, wherein the nucleic acid vector is a BAC.

5. The method of any one of claims 1 to 4, wherein the mammalian cell is a HEK 293 cell, CHO cell, Jurkat cell, KS62 cell, PerC6 cell, HeLa cell.

6. The method of claim 5, wherein the mammalian cell is a suspension adapted/nonadherent cell.

7. A method of producing a replication defective retroviral vector particle, comprising:
(a) introducing a nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid comprising retroviral nucleic acid sequences encoding:
gag and pol proteins, and
an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus,
wherein each of the nucleic acid sequences has its own promoter within the nucleic acid vector, into a culture of mammalian host cells;
(b) selecting within the culture for a mammalian host cell which has the nucleic acid sequences encoded on the vector integrated into an endogenous chromosome of the mammalian host cell; and
(c) further culturing the mammalian host cell under conditions in which the replication defective retroviral vector particle is produced.

8. The method of claim 7, additionally comprising isolating the replication defective retroviral vector particle.

9. A method according to any one of claims 1 to 8, wherein the retroviral nucleic acid sequences are derived from a retrovirus selected from lentivirus, alpha-retrovirus, gamma-retrovirus or foamy-retrovirus.

10. A method according to claim 9, wherein the retroviral nucleic acid sequences are derived from a lentivirus selected from the group consisting of HIV-1, HIV-2, SIV, FIV, EIAV and Visna.

11. A method according to claim 10, wherein the retroviral nucleic acid sequences are derived from HIV-1.

12. A method according to any one of claims 1 to 11, wherein the env protein is derived from Vesicular stomatitis virus.

13. A nucleic acid vector comprising a non-mammalian origin of replication, one or a plurality of recombination sites(s) and the ability to hold at least 25 kilobases (kb) of DNA selected from: a bacterial artificial chromosome (BAC), a yeast artificial chromosome (YAC), a P1-derived artificial chromosome, a fosmid or a cosmid comprising retroviral nucleic acid sequences encoding:
gag and pol proteins, and
an env protein obtained from or derived from a virus selected from a Vesiculovirus, Lyssavirus, Arenavirus, Alphavirus, Filovirus, Coronavirus, Respirovirus, Hepacivirus, Influenzavirus and Nucleopolyhedrovirus,
wherein each of the nucleic acid sequences has its own promoter within the nucleic acid vector.

14. A nucleic acid vector according to claim 13, which additionally comprises nucleic acid sequences which encode the RNA genome of a retroviral vector particle.

15. A nucleic acid vector according to any one of claims 13 to 14, which additionally comprises the auxiliary gene rev.

16. A nucleic acid vector according to any one of claims 13 to 15, which is a BAC.

## Patentansprüche

1. Verfahren zum Herstellen einer stabilen retroviralen Verpackungszelllinie, umfassend:
(a) Einführen eines Nukleinsäurevektors umfassend einen nicht von einem Säugetier stammenden Replikationsursprung, eine oder eine Vielzahl von Rekombinationsstelle(n) und die Fähigkeit mindestens 25 Kilobasen (kb) DNA zu halten ausgewählt aus: einem bakteriellen artifiziellen Chromosom (BAC), einem Hefe artifiziellen Chromosom (YAC), einem P1-abgeleiteten artifiziellen Chromosom, einem Fosmid oder einem Cosmid umfassend retrovirale Nukleinsäuresequenzen kodierend für:
Gag- und Pol-Proteine, und
ein Env-Protein, das von einem Virus erhalten oder abgeleitet wurde ausgewählt aus einem Vesiculovirus, Lyssavirus, Arenavirus, Alphavirus, Filovirus, Coronavirus, Respirovirus, Hepacivirus, Influenzavirus und Nukleopolyhedrovirus,
wobei jede der Nukleinsäuresequenzen ihren eigenen Promoter innerhalb des Nukleinsäurevektors hat,
in eine Kultur von Säugetierwirtszellen; und
(b) in der Kultur für eine Säugetierwirtszelle Selektieren, welche die auf dem Vektor kodierten Nukleinsäuresequenzen in ein endogenes Chromosom der Säugetierwirtszelle integriert hat.

2. Verfahren gemäß Anspruch 1, wobei der Nukleinsäurevektor zudem Nukleinsäuresequenzen, die für das RNA-Genom eines retroviralen Vektorpartikels kodieren, umfasst.

3. Verfahren gemäß Anspruch 1 oder 2, wobei der Nukleinsäurevektor zudem das zusätzliche Gen rev umfasst.

4. Verfahren gemäß irgendeinem der Ansprüche 1 bis 3, wobei der Nukleinsäurevektor ein BAC ist.

5. Verfahren gemäß irgendeinem der Ansprüche 1 bis 4, wobei die Säugetierzelle eine HEK293-Zelle, CHO-Zelle, Jurkat-Zelle, KS62-Zelle, PerC6-Zelle, HeLa-Zelle ist.

6. Verfahren gemäß Anspruch 5, wobei die Säugetierzelle eine an Suspension adaptierte / nicht-adhärente Zelle ist.

7. Verfahren zum Herstellen eines replikationsdefekten retroviralen Vektorpartikels, umfassend:
(a) Einführen eines Nukleinsäurevektors umfassend einen nicht von einem Säugetier stammenden Replikationsursprung, eine oder eine Vielzahl von Rekombinationsstelle(n) und die Fähigkeit mindestens 25 Kilobasen (kb) DNA zu halten ausgewählt aus: einem bakteriellen artifiziellen Chromosom (BAC), einem Hefe artifiziellen Chromosom (YAC), einem P1-abgeleiteten artifiziellen Chromosom, einem Fosmid oder einem Cosmid umfassend retrovirale Nukleinsäuresequenzen kodierend für:
Gag- und Pol-Proteine, und
ein Env-Protein, das von einem Virus erhalten oder abgeleitet wurde ausgewählt aus Vesiculovirus, Lyssavirus, Arenavirus, Alphavirus, Filovirus, Coronavirus, Respirovirus, Hepacivirus, Influenzavirus und Nukleopolyhedrovirus,
wobei jede der Nukleinsäuresequenzen ihren eigenen Promoter innerhalb des Nukleinsäurevektors hat, in eine Kultur von Säugetierwirtszellen;
(b) in der Kultur für eine Säugetierwirtszelle Selektieren, welche die auf dem Vektor kodierten Nukleinsäuresequenzen in ein endogenes Chromosom der Säugetierwirtszelle integriert hat; und
(c) weitergehend Kultivieren der Säugetierwirtszelle unter Bedingungen, unter denen das replikationsdefekte retrovirale Vektorpartikel hergestellt wird.

8. Verfahren gemäß Anspruch 7, weiter umfassend Isolieren des replikationsdefekten retroviralen Vektorpartikels.

9. Verfahren gemäß irgendeinem der Ansprüche 1 bis 8, wobei die retroviralen Nukleinsäuresequenzen von einem Retrovirus abgeleitet sind, der ausgewählt ist aus Lentivirus, Alpharetrovirus, Gammaretrovirus oder Foamy-Retrovirus.

10. Verfahren gemäß Anspruch 9, wobei die retroviralen Nukleinsäuresequenzen von einem Lentivirus ausgewählt aus der Gruppe bestehend aus HIV-1, HIV-2, SIV, FIV, EIAV und Visna abgeleitet sind.

11. Verfahren gemäß Anspruch 10, wobei die retroviralen Nukleinsäuresequenzen von HIV-1 abgeleitet sind.

12. Verfahren gemäß irgendeinem der Ansprüche 1 bis 11, wobei das Env-Protein von vesikulärem Stomatitis-Virus abgeleitet ist.

13. Nukleinsäurevektor umfassend einen nicht von einem Säugetier stammenden Replikationsursprung, eine oder eine Vielzahl von Rekombinationsstelle(n) und die Fähigkeit mindestens 25 Kilobasen (kb) DNA zu halten ausgewählt aus: einem bakteriellen artifiziellen Chromosom (BAC), einem Hefe artifiziellen Chromosom (YAC), einem P1-abgeleiteten artifiziellen Chromosom, einem Fosmid oder einem Cosmid umfassend retrovirale Nukleinsäuresequenzen kodierend für:
Gag- und Pol-Proteine, und
ein Env-Protein, das von einem Virus erhalten oder abgeleitet wurde ausgewählt aus Vesiculovirus, Lyssavirus, Arenavirus, Alphavirus, Filovirus, Coronavirus, Respirovirus, Hepacivirus, Influenzavirus und Nukleopolyhedrovirus,
wobei jede der Nukleinsäuresequenzen ihren eigenen Promoter innerhalb des Nukleinsäurevektors hat.

14. Nukleinsäurevektor gemäß Anspruch 13, welcher zudem Nukleinsäuresequenzen, die für das RNA-Genom eines retroviralen Vektorpartikels kodieren, umfasst.

15. Nukleinsäurevektor gemäß irgendeinem der Ansprüche 13 bis 14, welcher zudem das zusätzliche Gen rev umfasst.

16. Nukleinsäurevektor gemäß irgendeinem der Ansprüche 13 bis 15, welcher ein BAC ist.

## Revendications

1. Procédé de production d'une lignée cellulaire d'empaquetage rétroviral stable, comprenant:
(a) l'introduction d'un vecteur d'acide nucléique comprenant une origine de réplication non mammifère, un ou une pluralité de sites de recombinaison et la capacité de contenir au moins 25 kilobases (kb) d'ADN choisi parmi: un chromosome artificiel bactérien (BAC), un chromosome artificiel de levure (YAC), un chromosome artificiel dérivé de P1, un fosmide ou un cosmide comprenant des séquences d'acide nucléique rétrovirales codant:
les protéines gag et pol, et
une protéine env obtenue à partir de ou dérivée d'un virus choisi parmi un Vesiculovirus, Lyssavirus, Arenavirus, Alphavirus, Filovirus, Coronavirus, Respirovirus, Hepacivirus, virus influenza et virus de la polyédrose nucléaire,
dans lequel chacune des séquences d'acide nucléique a son propre promoteur dans le vecteur d'acide nucléique, dans une culture de cellules hôtes de mammifère; et
(b) la sélection dans la culture d'une cellule hôte de mammifère qui a les séquences d'acide nucléique codées sur le vecteur intégrées dans un chromosome endogène de la cellule hôte de mammifère.

2. Procédé selon la revendication 1, dans lequel le vecteur d'acide nucléique comprend en outre des séquences d'acide nucléique qui codent le génome à ARN d'une particule de vecteur rétroviral.

3. Procédé selon les revendications 1 ou 2, dans lequel le vecteur d'acide nucléique comprend en outre le gène auxiliaire rev.

4. Procédé selon l'une quelconque des revendications 1 à 3, dans lequel le vecteur d'acide nucléique est un BAC.

5. Procédé selon l'une quelconque des revendications 1 à 4, dans lequel la cellule de mammifère est une cellule HEK 293, une cellule CHO, une cellule Jurkat, une cellule KS62, une cellule PerC6, une cellule HeLa.

6. Procédé selon la revendication 5, dans lequel la cellule de mammifère est une cellule adaptée à la suspension/non adhérente.

7. Procédé de production d'une particule de vecteur rétroviral défectif pour la réplication, comprenant:
(a) l'introduction d'un vecteur d'acide nucléique comprenant une origine de réplication non mammifère, un ou une pluralité de sites de recombinaison et la capacité de contenir au moins 25 kilobases (kb) d'ADN choisi parmi: un chromosome artificiel bactérien (BAC), un chromosome artificiel de levure (YAC), un chromosome artificiel dérivé de P1, un fosmide ou un cosmide comprenant des séquences d'acide nucléique rétrovirales codant:
les protéines gag et pol, et
une protéine env obtenue à partir de ou dérivée d'un virus choisi parmi un Vésiculovirus, Lyssavirus, Arenavirus, Alphavirus, Filovirus, Coronavirus, Respirovirus, Hepacivirus, virus influenza et virus de la polyédrose nucléaire,
dans lequel chacune des séquences d'acide nucléique a son propre promoteur dans le vecteur d'acide nucléique dans une culture de cellules hôtes de mammifère;
(b) la sélection dans la culture d'une cellule hôte de mammifère qui a les séquences d'acide nucléique codées sur le vecteur intégrées dans un chromosome endogène de la cellule hôte de mammifère; et
(c) la culture continuée de la cellule hôte de mammifère dans des conditions dans lesquelles la particule de vecteur rétroviral défectif pour la réplication est produite.

8. Procédé selon la revendication 7, comprenant en outre l'isolement de la particule de vecteur rétroviral défectif pour la réplication.

9. Procédé selon l'une quelconque des revendications 1 à 8, dans lequel les séquences d'acide nucléique rétrovirales sont dérivées d'un rétrovirus choisi parmi les lentivirus, alpha-rétrovirus, gamma-rétrovirus ou rétrovirus mousseux.

10. Procédé selon la revendication 9, dans lequel les séquences d'acide nucléique rétrovirales sont dérivées d'un lentivirus choisi dans le groupe consistant en VIH-1, VIH-2, SIV, FIV, EIAV et Visna.

11. Procédé selon la revendication 10, dans lequel les séquences d'acide nucléique rétrovirales sont dérivées de VIH-1.

12. Procédé selon l'une quelconque des revendications 1 à 11, dans lequel la protéine env est dérivée du virus de la stomatite vésiculeuse.

13. Vecteur d'acide nucléique comprenant une origine de réplication non mammifère, un ou une pluralité de sites de recombinaison et la capacité de contenir au moins 25 kilobases (kb) d'ADN choisi parmi: un chromosome artificiel bactérien (BAC), un chromosome artificiel de levure (YAC), un chromosome artificiel dérivé de P1, un fosmide ou un cosmide comprenant des séquences d'acide nucléique rétrovirales codant:
les protéines gag et pol, et
une protéine env obtenue à partir de ou dérivée d'un virus choisi parmi un Vesiculovirus, Lyssavirus, Arenavirus, Alphavirus, Filovirus, Coronavirus, Respirovirus, Hepacivirus, virus influenzavirus et virus de la polyédrose nucléaire, dans lequel chacune des séquences d'acide nucléique a son propre promoteur dans le vecteur d'acide nucléique.

14. Vecteur d'acide nucléique selon la revendication 13, qui comprend en outre des séquences d'acide nucléique qui codent le génome à ARN d'une particule de vecteur rétroviral.

15. Vecteur d'acide nucléique selon l'une quelconque des revendications 13 à 14, qui comprend en outre le gène auxiliaire rev.

16. Vecteur d'acide nucléique selon l'une quelconque des revendications 13 à 15, qui est un BAC.
